(19) Europäisches Patentamt
European Patent Office
Office européen des brevets

(11) **EP 2 365 954 B1**

(12) **FASCICULE DE BREVET EUROPEEN**

(45) Date de publication et mention
de la délivrance du brevet:
**07.11.2018 Bulletin 2018/45**

(21) Numéro de dépôt: **09802191.8**

(22) Date de dépôt: **16.12.2009**

(51) Int Cl.:
*C07C 45/52* (2006.01)     *C07C 47/22* (2006.01)
*C07C 319/18* (2006.01)     *C07C 323/22* (2006.01)
*C07C 323/52* (2006.01)     *C07C 323/58* (2006.01)
*B01J 38/12* (2006.01)     *B01J 23/20* (2006.01)
*B01J 23/92* (2006.01)

(86) Numéro de dépôt international:
**PCT/FR2009/052577**

(87) Numéro de publication internationale:
**WO 2010/076510 (08.07.2010 Gazette 2010/27)**

(54) **PROCEDE DE PREPARATION D'ACROLEINE A PARTIR DE GLYCEROL OU DE GLYCERINE**

VERFAHREN ZUR HERSTELLUNG VON ACROLEIN AUS GLYCERIN

METHOD FOR PREPARING ACROLEIN FROM GLYCEROL OR GLYCERINE

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **16.12.2008 FR 0858624**

(43) Date de publication de la demande:
**21.09.2011 Bulletin 2011/38**

(73) Titulaires:
• **Adisseo France S.A.S.**
**92160 Antony (FR)**
• **Centre National de la Recherche Scientifique
(C.N.R.S.)**
**75794 Paris Cedex 16 (FR)**

(72) Inventeurs:
• **BELLIERE-BACA, Virginie**
**F-78570 Andresy (FR)**
• **LORIDANT, Stéphane**
**F-69330 Meyzieu (FR)**
• **MILLET, Jean-Marc**
**F-69006 Lyon (FR)**
• **LAURIOL-GARBEY, Pascaline**
**F-69009 Lyon (FR)**

(74) Mandataire: **Verriest, Philippe et al
Cabinet Germain & Maureau
12, rue Boileau
69006 Lyon (FR)**

(56) Documents cités:
WO-A-2006/087083     WO-A-2008/066079
JP-A- 2007 268 363     JP-A- 2008 266 165
US-A1- 2008 183 013     US-A1- 2008 214 384

• S. Vives ET AL: "Sol-gel/co-precipiation method
for the preparation and characterization of
zirconia-tungsten composite powders", Journal
of Materials Science, vol. 34, no. 13, 1 January
1999 (1999-01-01), pages 3127-3135,
XP055342242, Dordrecht ISSN: 0022-2461, DOI:
10.1023/A:1004661202300
• ROSS-MEDGAARDEN E I ET AL: "New insights
into the nature of the acidic catalytic active sites
present in ZrO"2-supported tungsten oxide
catalysts", JOURNAL OF CATALYSIS,
ACADEMIC PRESS, DULUTH, MN, US, vol. 256,
no. 1, 15 May 2008 (2008-05-15), pages 108-125,
XP022655702, ISSN: 0021-9517, DOI:
10.1016/J.JCAT.2008.03.003 [retrieved on
2008-04-10]

**Description**

La présente invention concerne un procédé catalytique de fabrication d'acroléine par déshydratation du glycérol ou glycérine et les applications d'un tel procédé.

On entend par glycérol, un glycérol purifié ou non, de préférence issu de la biomasse, et notamment un glycérol hautement purifié ou partiellement purifié. Un glycérol purifié possède une pureté supérieure ou égale à 98%, obtenu par distillation de glycérine. Un glycérol non purifié ou seulement partiellement purifié pourra être en solution dans du méthanol lorsqu'il provient par exemple d'une transestérification de triglycérides, comme décrit ci-après. On entend par glycérine, notamment, une glycérine d'origine naturelle, issue de l'hydrolyse d'huiles végétales et/ou de graisses animales, ou une glycérine d'origine synthétique, issue du pétrole, plus ou moins purifiée ou raffinée, ou bien brute. A titre d'exemple, une glycérine brute a un titre compris entre 80 et 85%. Ainsi, dans la suite de la description, on se réfère principalement à la conversion d'un glycérol ou d'une glycérine issue de la biomasse, mais l'invention n'y est bien entendu pas limitée et son intérêt s'étend à tous glycérols et glycérines, quels que soient leurs origines et leurs degrés de pureté.

L'épuisement progressif des énergies fossiles conduit les industriels à envisager l'utilisation de matières premières renouvelables issues de la biomasse pour la production de carburants. Dans ce contexte, le biodiesel est un carburant produit à partir d'huile végétale ou animale.

Ce produit jouit d'une aura verte en raison d'un bilan $CO_2$ nettement favorable par rapport aux énergies fossiles. Le diester® (ou EMVH, Esters Méthyliques d'Huiles Végétales) est un biodiesel fabriqué par transestérification des triglycérides présents dans les liquides oléagineux, notamment les huiles végétales de palme, colza et tournesol, par du méthanol. Cette transestérification coproduit approximativement, et suivant les procédés envisagés, 100 kg de glycérol par tonne de diester®. La partie non lipidique de la matière première utilisée, les tourteaux, est principalement mise à profit dans l'alimentation animale.

Ce biodiesel est utilisé en mélange dans le gazole. Les directives européennes 2001/77/EC et 2003/30/EC, qui seront appliquées dans un futur proche, projettent d'introduire 7% en 2010 et 10% à l'horizon 2015 de diester® dans les gazoles. Cette augmentation substantielle de la quantité de biodiesel produit va générer des quantités importantes de glycérol équivalentes à plusieurs centaines de milliers de tonnes/an.

Quelques 1500 utilisations du glycérol sont déjà répertoriées, parmi lesquelles les suivantes illustrent à titre d'exemples sa présence dans de nombreuses et diverses formulations :

- hydratants en pharmacie (dans les suppositoires et les sirops) ou en cosmétologie dans les crèmes hydratantes, les savons à la glycérine, les dentifrices,
- solvants dans l'industrie alimentaire,
- plastifiants ou lubrifiants dans l'industrie chimique.

Ces applications s'avèreront nettement insuffisantes pour absorber les quantités de glycérol qui seront coproduites avec les biodiesels et bien qu'en progression, le marché conventionnel du glycérol (savons, pharmacie, ...) ne pourra pas non plus absorber un tel surplus. Il est donc vital de trouver de nouvelles applications permettant de valoriser de très gros volumes de glycérol.

Devant ce constat, de nombreux débouchés ont été étudiés ces dernières années (voir M. Pagliaro et al., Angew. Chem. Int. Ed. (2007) 46, 4434 - 4440 ainsi que M. Pagliaro, M. Rossi: The Future of Glycerol, RSC Publishing, Cambridge (2008)), avec, en particulier, les six voies de valorisation suivantes :

- conversion en 1,3-propanediol et en 1,2-propanediol, notamment utilisés comme monomères de base dans la synthèse des polyesters et polyuréthanes,
- conversion en monoesters pour la chimie des lubrifiants,
- conversion en polyglycérols employés en tant qu'agents émulsifiants, additifs alimentaires,
- conversion en acroléine (par déshydratation) et acide acrylique (par déshydratation et oxydation),
- valorisation directe en tant qu'additifs pour l'alimentation animale.

L'acroléine et l'acide acrylique sont traditionnellement produits par oxydation ménagée en phase gazeuse du propylène par l'oxygène de l'air en présence de catalyseurs à base d'oxydes de molybdène et/ou bismuth. L'acroléine ainsi obtenue peut soit être directement intégrée dans un procédé en deux étapes de fabrication d'acide acrylique, soit être utilisée comme intermédiaire de synthèse. La production de ces deux monomères est donc étroitement liée au propylène qui est, en substance, fabriqué par vapocraquage ou craquage catalytique de coupes pétrolières.

Les marchés de l'acroléine, l'un des plus simples des aldéhydes insaturés, et de l'acide acrylique sont gigantesques car ces monomères entrent dans la composition de nombreux produits de masse.

Par ailleurs, l'acroléine, composé hautement réactif de par sa structure, trouve de nombreuses applications, notamment comme intermédiaire de synthèse. Elle est tout particulièrement utilisée comme intermédiaire clé entrant dans la synthèse de la D,L-méthionine et de son dérivé hydroxy-analogue, l'acide 2-hydroxy-4méthylthiobutanoïque (HMTBA). Ces additifs alimentaires sont massivement employés car ils entrent dans la composition de compléments alimentaires indispensables à la croissance des animaux (volailles, porcs, ruminants, poissons, ...). Dans un certain nombre de cas, il peut être profitable de pouvoir augmenter, voire d'assurer les capacités de production des unités industrielles existantes en diversifiant la matière première engagée. Il apparaît donc tout particulièrement intéressant de pouvoir augmenter la productivité en acroléine, tout en réduisant la dépendance vis-à-vis de cette ressource issue du pétrole qu'est le propylène.

L'objet de la présente invention réside dans la mise en oeuvre de catalyseurs robustes, actifs, sélectifs et régénérables, permettant de produire de l'acroléine directement à partir de glycérol ou de glycérine, notamment issus de la biomasse, selon la réaction :

$$HO\text{-}CH_2\text{-}CH(OH)\text{-}CH_2\text{-}OH \rightarrow CH_2\text{=}CH\text{-}CHO + 2H_2O$$

Cette alternative permet ainsi de disposer d'un procédé compétitif de synthèse d'acroléine non dépendant de la ressource pétrolière propylène à partir d'une autre matière première renouvelable.

Cette possibilité est particulièrement avantageuse pour synthétiser la méthionine ou ses analogues, comme son hydroxy-analogue (HMTBA), directement à partir de la biomasse.

Ainsi, l'invention se rapporte en outre à une application de cette réaction à la synthèse de l'aldéhyde-3-(méthylthio)propionique (MMP), du 2-hydroxy-4-méthylthiobutyronitrile (HMTBN), de la méthionine et ses analogues tels que l'acide 2-hydroxy-4méthylthiobutanoïque (HMTBA), les esters du HMTBA comme l'ester isopropylique, l'acide 2-oxo-4-méthylthiobutanoïque, à partir d'acroléine.

La méthionine, le HMTBA et les esters de celui-ci et analogues, sont utilisés en nutrition animale et, dans leurs procédés industriels de synthèse, l'acroléine est généralement obtenue par oxydation du propylène et/ou du propane. L'oxydation du propylène en acroléine par l'air en présence d'eau est partielle, et le produit brut résultant, à base d'acroléine, contient aussi du propylène et du propane n'ayant pas réagi, de l'eau et des sous-produits de la réaction d'oxydation, notamment des acides, aldéhydes et alcools.

Le glycérol (appelé aussi glycérine) est depuis longtemps connu comme une source d'acroléine (transformation thermique), c'est un produit que l'on trouve largement dans la nature, sous forme d'esters (triglycérides), en particulier dans toutes les huiles et graisses animales ou végétales, ce qui en fait un réactif de départ disponible en quantité et en cela utilisable industriellement. Il est effectivement connu que le glycérol se décompose pour donner de l'acroléine lorsqu'il est porté à des températures supérieures à 280°C. Cette réaction faiblement sélective s'accompagne de la formation de nombreux sous-produits dont l'acétaldéhyde, l'hydroxyacétone, en plus des produits d'oxydation totale $CO$, $CO_2$. Il est donc indispensable de contrôler la réaction de transformation du glycérol en acroléine pour éviter un gaspillage inutile de cette ressource et s'affranchir d'une séparation postérieure énergétiquement coûteuse avec un procédé de purification de l'acroléine complexe. Par ailleurs, ces impuretés, principalement les dérivés aromatiques, sont souvent à l'origine de formation de coke à la surface du catalyseur qui empoisonne ce dernier au cours du temps; il est souvent nécessaire de régénérer le catalyseur de façon à retrouver une activité catalytique satisfaisante.

De nombreux chercheurs universitaires et industriels se sont penchés sur cette réaction. Il a notamment été envisagé d'utiliser de l'eau supercritique comme milieu réactionnel. L'utilisation de solvant supercritique à l'échelle industrielle demeure difficile pour un procédé continu en raison d'infrastructures particulièrement lourdes qui exigent des autoclaves fonctionnant sous très haute pression. En revanche, la mise en place d'un procédé continu ou discontinu devient envisageable si un système catalytique performant, sélectif et résistant est identifié.

Devant l'intérêt grandissant de cette alternative chimique, la littérature fait état d'un grand nombre d'études relatives à l'utilisation des systèmes catalytiques basés sur des hétéropolyacides phospho- ou silico-tungstiques supportés, des oxydes mixtes et des zéolithes, utilisables pour des procédés continus ou discontinus en phase liquide ou gazeuse.

Ainsi, les documents WO-A-2006087083 et WO-A-2006087084 décrivent un procédé de déshydratation catalytique de glycérol en acroléine en phase gazeuse, en présence d'oxygène moléculaire et d'un catalyseur fortement acide choisi parmi des zéolites, le Nafion®, des oxydes de métaux choisis parmi l'aluminium, le zirconium, le titane, le niobium, le tantale, le silicium, imprégnés par des fonctions acides sous forme de groupements sulfates, borates, tungstates, silicates et phosphates.

Le document WO-A-2007132926 divulgue un procédé de conversion du glycérol en acroléine en présence d'un catalyseur choisi parmi des métallosilicates cristallins acides tels que des zéolithes de type structural MFI ou BEA, comprenant du silicium et un élément de préférence choisi parmi Al, Fe et Ga.

WO2008/066079A décrit un procédé de fabrication d'acide acrylique à partir de glycérol, en une seule étape, en utilisant un catalyseur comprenant à titre principal du molybdène. De même, US2008/183013A1 concerne la conversion du glycérol en acide acrylique, l'acroléine étant un intermédiaire non isolé.

US2008/0214384A1 se rapporte à un procédé de régénération d'un catalyseur de conversion du glycérol en acroléine, ce catalyseur étant acide et à base de tungstène, après avoir servi dans la réaction de déshydratation du glycérol en acroléine où il a perdu de l'activité et/ou de la sélectivité.

Les documents JP2007/268363A et JP2008/266165A décrivent l'utilisation de mélanges d'oxydes pour catalyser la réaction de conversion du glycérol en acroléine.

Par rapport aux procédés connus, on apporte, selon l'invention décrite, un procédé de préparation d'acroléine à partir de glycérol ou de glycérine, par déshydratation catalytique du glycérol en présence d'un catalyseur qui, tout en permettant de convertir la totalité du glycérol de départ, à la fois peut être très facilement régénéré et possède une longue durée de vie. Les auteurs de l'invention ont découvert que ce catalyseur était à base d'oxyde de zirconium et consistait en au moinsun oxyde mixte de zirconium et d'au moins un métal M, le dit métal étant le niobium.

Ainsi, l'invention concerne un procédé d'obtention d'acroléine à partir de glycérol ou de glycérine, en présence d'un catalyseur tel que défini ci-dessus, et l'utilisation de ce catalyseur, pour convertir du glycérol ou de la glycérine en acroléine. Un catalyseur de l'invention permet une conversion contrôlée du glycérol ou de la glycérine en acroléine, c'est-à-dire ne favorisant pas la conversion jusqu'à l'acide acrylique. A cet effet, un catalyseur préféré de l'invention ne comprend pas, ou ne comprend pas en proportion majoritaire en poids par rapport à chacun des autres oxydes le constituant, d'oxyde de molybdène et/ou d'oxyde de cuivre.

C'est pourquoi l'invention concerne aussi l'utilisation d'au moins un catalyseur, tel que défini précédemment pour convertir du glycérol ou de la glycérine en acroléine.

Le catalyseur peut être préparé de diverses façons (coprécipitation, synthèse hydrothermale...). Une procédure efficace a été décrite par Kantcheva. et al., Catalysis Communications (2008), 9(5), p874-879, dans les brevets FR 2907444 et FR 2907445.

Le catalyseur défini précédemment peut en outre répondre aux caractéristiques préférentielles ci-dessous, considérées seules ou en combinaison :

- le catalyseur n'est constitué que des oxydes et oxydes mixtes précédemment définis,
- au moins l'un des oxydes, mixtes ou non, desdits catalyseurs est supporté,
- le rapport molaire Zr/somme des autres éléments constitutifs desdits catalyseurs a) à f) différents de Zr, c'est-à-dire choisis parmi Si, Ti et M, varie de 0,5 à 200, plus avantageusement, il varie de 1 à 100.

Comme dit précédemment, le catalyseur de l'invention présente l'intérêt de pouvoir être régénéré facilement, et ceci sans que le rendement de la déshydratation, ni la sélectivité en acroléine ne soient affectés.

La réaction selon l'invention peut être mise en oeuvre en phase gazeuse ou en phase liquide, de préférence en phase gazeuse. Lorsque la réaction est menée en phase gazeuse, différentes technologies de procédé peuvent être utilisées, à savoir procédé en lit fixe, procédé en lit fluidisé ou procédé en lit fluidisé circulant. Dans les deux premiers procédés, en lit fixe ou en lit fluidisé, la régénération du catalyseur peut être séparée de la réaction catalytique. Elle peut par exemple se faire *ex situ* par les méthodes de régénération conventionnelles, comme la combustion sous air ou avec un mélange gazeux contenant de l'oxygène moléculaire. Selon le procédé de l'invention, la régénération peut se faire *in situ* car les températures et pressions auxquelles se fait la régénération sont voisines des conditions réactionnelles du procédé.

S'agissant du procédé en phase liquide, la réaction peut être réalisée dans un réacteur classique pour réaction en phase liquide sur un catalyseur solide, mais aussi dans un réacteur de type distillation catalytique en égard à la différence significative des points d'ébullition du glycérol (290°C) et de l'acroléine (53°C). On peut également raisonnablement envisager un procédé en phase liquide à une température relativement basse qui permet une distillation continue de l'acroléine produite, limitant ainsi les réactions consécutives de dégradation de l'acroléine. Les conditions expérimentales de la réaction en phase gazeuse sont de préférence une température comprise entre 250 et 400°C à une pression comprise entre 1 et 10 bars. En phase liquide, la réaction est opérée entre 150 et 350°C et à une pression pouvant aller de 3 à 70 bars.

Un autre avantage du procédé de l'invention réside dans la forme du glycérol ou glycérine de départ qui peut être sous forme pure ou partiellement purifiée ou en solution, notamment aqueuse. Avantageusement, on utilise une solution aqueuse de glycérol. En solution aqueuse, la concentration du glycérol est de préférence d'au moins 1%, au mieux elle varie de 10 à 50% en poids et de préférence entre 15 et 30% en poids dans le réacteur. La concentration en glycérol ne doit pas être trop élevée dans le but d'éviter les réactions parasites qui grèvent le rendement en acroléine, comme la formation des éthers de glycérol ou des réactions d'acétalisation entre l'acroléine produite et le glycérol non converti. Par ailleurs, la solution de glycérol ne doit pas être trop diluée, en raison d'un coût énergétique rédhibitoire induit par l'évaporation du glycérol. Dans tous les cas, il est aisé d'ajuster la concentration de la solution de glycérol en recyclant partiellement ou totalement l'eau produite par la réaction considérée. L'optimisation énergétique aux bornes de la synthèse tend à récupérer la chaleur en sortie de réaction pour vaporiser le flux de glycérol alimenté au réacteur.

Un autre objet de l'invention est un procédé de fabrication de l'aldéhyde-3-(méthylthio)propionique (MMP), du 2-hydroxy-4-méthylthiobutyronitrile (HMTBN), de la méthionine, de l'acide 2-hydroxy-4-méthylthiobutanoïque (HMTBA), des esters de ce dernier, notamment l'ester isopropylique, et l'acide 2-oxo-4-méthylthiobutanoïque (KMB) à partir d'acroléine, selon lequel l'acroléine est obtenue par un procédé décrit ci-dessus. Comparativement au procédé conventionnel de fabrication de l'acroléine par l'oxydation ménagée du propylène, l'acroléine produite selon le procédé susmentionné peut contenir des impuretés différentes du procédé traditionnel, tant sous l'angle de leur quantité que de leur nature. Selon l'utilisation envisagée, synthèse de l'acide acrylique ou de la méthionine ou son hydroxyanalogue, il pourra être envisagé de purifier l'acroléine selon les techniques connues de l'homme de l'art.

Ainsi, une fois l'acroléine directement obtenue selon l'invention ou après purification, elle est mise en réaction avec du méthylmercaptan (MSH) pour produire l'aldéhyde-3-(méthylthio)propionique (ou MMP). Dans une étape suivante, le MMP est mis en contact avec de l'acide cyanhydrique pour produire le 2-hydroxy-4-(méthylthio)butyronitrile (HMTBN). Après synthèse du HMTBN, diverses étapes réactionnelles conduisent à la méthionine, son hydroxyanalogue (HMTBA), les esters de ce dernier, ou son oxoanalogue (KMB). Toutes ses étapes à compter de la synthèse de l'acroléine sont bien connues de l'homme du métier.

La présente invention est maintenant décrite plus en détail et illustrée avec les exemples et figures ci-après sans toutefois en limiter la portée.

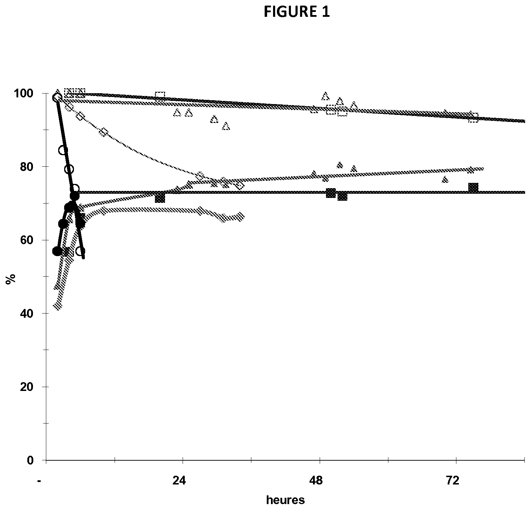

La Figure 1 présente l'évolution de la conversion en glycérol et de la sélectivité en acroléine correspondante au cours du temps, sur chacun des catalyseurs A, B, C et D décrits dans les exemples 1, 7, 8 et 9 respectivement; le catalyseur A est un catalyseur de l'invention, le catalyseur B est hors invention, les catalyseurs C et D sont des catalyseurs de l'art antérieur. Le temps indiqué pour chaque point est celui de la fin d'un prélèvement correspondant à un piégeage pendant une heure. Les conditions réactionnelles et les méthodes de calcul utilisées par la conversion et la sélectivité en acroléine sont décrites plus loin.

Cette figure se lit à l'appui de la légende suivante:

- conversion en glycérol sur catalyseur A(□), B(△), C(◇) ou D(○)
- sélectivité en acroléine sur catalyseur A(■), B(▲), C(♦) ou D(●)

La Figure 2 illustre la conversion en glycérol et la sélectivité en acroléine obtenues sur le catalyseur A selon l'invention avant et après régénération sous flux d'air.

Cette figure se lit à l'appui de la légende suivante :

- conversion en glycérol avec catalyseur frais (△) et avec catalyseur régénéré (▲)
- sélectivité en acroléine avec catalyseur frais (□) et avec catalyseur régénéré (■)

La Figure 3 représente une comparaison de la conversion en glycérol et de la sélectivité en acroléine de cette conversion au cours du temps, avec chacun des catalyseurs A', C et D décrits dans les exemples 2, 8 et 9 respectivement; le catalyseur A' est un catalyseur de l'invention, les catalyseurs C et D, des catalyseurs de l'art antérieur.

Cette figure se lit à l'appui de la légende suivante :

- conversion en glycérol avec catalyseur A'(♦), D(●) ou C(△)
- sélectivité en acroléine avec catalyseur A'(■), D(x) ou C(▲)

La Figure 4 illustre la conversion en glycérol et la sélectivité en acroléine obtenues avec le catalyseur A' selon l'invention avant et après régénération sous flux d'air.

Cette figure se lit à l'appui de la légende suivante :

- conversion en glycérol avec catalyseur frais (△) et avec catalyseur régénéré (▲)
- sélectivité en acroléine avec catalyseur frais (□) et avec catalyseur régénéré (■)

Les conditions réactionnelles et les méthodes de calcul de la conversion et de la sélectivité en acroléine sont décrites ci-après.

La réaction de déshydratation du glycérol est conduite sur les catalyseurs indiqués, à pression atmosphérique, dans un réacteur droit à lit fixe de diamètre 18mm. Le réacteur est placé dans un four qui permet de maintenir le catalyseur à la température de réaction qui est de 300°C. Le volume de catalyseur chargé dans le réacteur est de 4,5 mL, ce qui donne une hauteur de lit d'environ 1,8 cm. Le réacteur est alimenté avec un débit de 3,77g/h de solution aqueuse à 20% en poids de glycérol. La solution aqueuse de glycérol est vaporisée grâce à un évaporateur C.E.M (Controlled Evaporator Mixer) Bronkhorst® en présence d'un débit d'azote de 75 mL/min. La proportion relative molaire glycérol/eau/azote est de 2,3/ 46,3/ 51,4. Le temps de contact calculé est de l'ordre de 1,9 s soit une GHSV de 1930 h$^{-1}$. Le temps de contact est défini comme suit :

$$\text{Temps de contact} = \text{Volume catalyseur} \times P_{atm} / (\text{débit molaire total} \times \text{Température} \times R)$$

avec $P_{atm}$=101325 Pa, Température =25°C et le débit molaire total = débit molaire du glycérol + débit molaire de l'eau + débit molaire du gaz inerte

Après réaction, les produits sont condensés. Deux systèmes de condensations ont été utilisés. Les exemples 10, 11, 12, 16 ,17 et 18 ont été obtenus avec un système de trois pièges montés en série. Le premier piège contient une masse connue d'eau et est réfrigéré par de la glace pilée. Les deux autres pièges contiennent de l'éthanol et sont refroidis par un cryostat à -25°C. Les exemples 13, 14 et 15 ont été obtenus avec un simple piège contenant une masse connue d'eau et réfrigéré par de la glace pilée. La durée de piégeage est d'une heure et le débit d'alimentation n'est pas interrompu pendant les changements de pièges.

Les produits formés sont analysés par chromatographie, deux analyses sont réalisées pour chaque prélèvement :

- Les principaux produits de la réaction sont analysés par chromatographie gazeuse sur une colonne capillaire (Nukol, 30m x 0,53 mm) avec un chromatographe Shimadzu 2014 muni d'un détecteur FID. Les produits quantifiés lors de cette analyse sont : l'acroléine, l'acétaldéhyde, l'acétone, le propionaldéhyde, l'hydroxypropanone, l'acide acétique, l'alcool allylique et le phénol ;
- Le glycérol restant est quantifié par chromatographie gazeuse avec un chromatographe Helwett Packard équipé d'un détecteur FID et d'une colonne capillaire (Carbowax ou ZBwax, 30 m x 0,32 mm).

La conversion en glycérol, la sélectivité en acroléine et les rendements en différents produits sont définis comme suit :

$$\text{Conversion en glycérol (\%)} = 100 \times (1 - \text{nombre de moles de glycérol restantes/nombre de moles de glycérol introduites})$$

$$\text{Sélectivité en acroléine (\%)} = 100 \times (\text{nombre de moles d'acroléine produites /nombre de moles de glycérol ayant réagi})$$

$$\text{Rendement en X (\%)} = K \times 100 \times \text{nombre de moles de X produites / nombre de moles de glycérol introduites}$$

Avec K= 1 si X est l'acroléine, l'acétone, l'hydroxypropanone, le propanal ou l'alcool allylique; K=2/3 si X= acétaldéhyde ou acide acétique et K=2 si X= phénol.

Exemple 1 : préparation et caractérisation du catalyseur A

Un catalyseur selon l'invention de type oxyde de zirconium et niobium préparé à partir d'oxyde de zirconium hydraté et d'oxalato-niobiate d'ammonium, $(NH_4)(C_2O_4)_2NbO.xH_2O$ (Aldrich, 99.99%). L'oxyde de zirconium hydraté est préparé par co-précipitation d'une solution d'oxonitrate de zirconium $ZrO(NO_3)_2.xH_2O$ (Aldrich, 99%) et d'une solution d'ammoniaque à 28% à pH=8,8.

L'oxalato-niobiate d'ammonium est dissous dans de l'eau permutée acidifiée avec $HNO_3$ concentré à pH~0,5 et chauffée à 45°C. Après retour à température ambiante, l'hydroxyde de zirconium hydraté est ajouté dans un rapport molaire $ZrO_2/Nb_2O_5$ de 3 :1, le degré d'hydratation de l'oxyde de zirconium hydraté est préalablement déterminé par analyse thermogravimétrique (ATG). Après 24h sous agitation le mélange est filtré et le solide calciné sous flux d'air à 600°C. La surface spécifique de ce catalyseur est de 40m$^2$/g. Les surfaces spécifiques des solides ont été mesurées par la méthode BET (Brunauer Emmet et Teller) à -196°C sur un appareil Micromeritics ASAP 2020. Les solides sont préalablement désorbés à 300°C pendant 3 heures sous un vide de 5x10$^{-5}$ mbar. Les teneurs en niobium et en zircone des différents solides préparés ont été déterminées par ICP-OES (Spectrométrie d'Emission Optique à plasma). Le rapport molaire Zr/Nb du catalyseur A calculé à partir de ces analyses est de 9,3.

Exemple 2 : préparation et caractérisation du catalyseur A'

Un catalyseur selon l'invention de type oxyde de zirconium et niobium est préparé selon la procédure décrite par Kantcheva. et al., Catalysis Communications (2008), 9(5), p874-879, par imprégnation d'oxyde de zirconium hydraté.

L'oxyde de zirconium hydraté a été préparé par co-précipitation d'une solution d'oxonitrate de zirconium $ZrO(NO_3)_2.xH_2O$ (Aldrich, 99%) et d'une solution d'ammoniaque à 28%. Le précurseur de Nb(V), $(NH_4)(C_2O_4)_2NbO.xH_2O$ (Aldrich, 99.99%), est ajouté sous agitation à une solution d'eau oxygénée à 35% (Sigma Aldrich) acidifiée à pH≈0,5 avec $HNO_3$ concentré et chauffée à 50°C. Le rapport molaire $H_2O_2$/oxalate est de 13/1. La solution est chauffée 1h à 50°C avant d'être refroidie à température ambiante. Puis, l'oxyde de zirconium hydraté est rajouté en assurant un ratio $ZrO_2$:$Nb_2O_5$ = 6:1, le degré d'hydratation de l'oxyde de zirconium hydraté étant déterminé par analyse thermogravimétrique (ATG). Le mélange est laissé sous agitation à température ambiante pendant 24h puis la phase liquide est évaporée sous vide à T<70°C. Le solide obtenu est calciné sous flux d'air à 600°C.

La surface spécifique de ce catalyseur est de 51 $m^2$/g. Les surfaces spécifiques des solides ont été mesurées par la méthode BET (Brunauer Emmet et Teller) à -196°C sur un appareil Micromeritics ASAP 2020. Les solides sont préalablement désorbés à 300°C pendant 3 heures sous un vide de $5 \times 10^{-5}$ mbar. Les teneurs en niobium et zirconium du solide obtenu ont été déterminées par ICP-OES. Le rapport molaire Zr/Nb de ce solide est de 3,3.

Exemple 3 : préparation et caractérisation du catalyseur E

Un catalyseur selon l'invention de type oxyde de zirconium et niobium est préparé selon la procédure décrite par Kantcheva. et al., (Catalysis Communications 9(5) (2008) p874-879) par imprégnation d'oxyde de zirconium hydraté par une solution contenant un oxalate mixte d'ammonium et de niobium.

Le précurseur de Nb(V), $(NH_4)(C_2O_4)_2NbO.xH_2O$ (Aldrich, 99.99%), est ajouté sous agitation à une solution d'eau oxygénée à 35% (Sigma Aldrich) acidifiée à pH~0,5 avec $HNO_3$ concentré et chauffée à 50°C. Le rapport molaire $H_2O_2$/oxalate est de 13/1. La solution est chauffée 1h à 50°C avant d'être refroidie à température ambiante. Puis, l'oxyde de zirconium hydraté, préalablement préparé par co-précipitation d'une solution d'oxonitrate de zirconium $ZrO(NO_3)_2.xH_2O$ (Aldrich, 99%) et d'une solution d'ammoniaque à 28%, est ajouté en assurant un ratio $ZrO_2$:$Nb_2O_5$ = 6:1. Le mélange est maintenu sous agitation à température ambiante pendant 24h puis la phase liquide est évaporée sous vide à T<70°C. Le solide obtenu est calciné sous flux d'air à 600°C.

La surface spécifique de ce catalyseur déterminée de façon similaire à celle du catalyseur A est de 39 $m^2$/g. Les teneurs en niobium et zirconium du solide obtenu ont été déterminées par ICP-OES. Le rapport molaire Zr/Nb de ce solide est de 3,7.

Exemple 4 : préparation et caractérisation du catalyseur F

Un catalyseur selon l'invention de type oxyde de zirconium, niobium et vanadium. Le précurseur de vanadium a été préparé à partir de $NH_4VO_3$ (Sigma, ACS Reagent 99,7%) selon la méthode suivante :
Le métavanadate d'ammonium est dissous dans une solution d'eau oxygénée à 9 % contenant de l'acide oxalique (Aldrich, 99%). Le rapport molaire acide oxalique/ $NH_4VO_3$ introduit est de 1,3. Après 1h sous agitation à température ambiante, la solution est évaporée sous vide ; un solide bleu est obtenu. La teneur en oxyde de vanadium de ce composé est déterminée par analyse thermogravimétrique.

Le précurseur de vanadium, l'oxalate mixte de niobium et ammonium $(NH_4)(C_2O_4)_2NbO.xH_2O$ (Aldrich, 99.99%), et l'oxyde de zirconium hydraté préparé comme décrit dans l'exemple 1 sont introduits dans une solution aqueuse acidifiée avec $HNO_3$ concentré (pH<0,5) avec un rapport molaire Zr/Nb/V de 72/22/3,2. Après 24h sous agitation, le mélange réactionnel est filtré et le solide calciné sous flux d'air à 600°C. La surface spécifique de ce catalyseur déterminée de façon similaire à celle du catalyseur A est de 48 $m^2$/g. Les teneurs en niobium, vanadium et zirconium du solide obtenu ont été déterminées par ICP-OES. La composition molaire Zr/Nb/V de ce catalyseur est 90,4/8,4/1,2.

Exemple 5 : préparation et caractérisation du catalyseur G (hors invention)

Un catalyseur de type zircone tungstée dopée à la silice. La préparation de ce solide comporte trois étapes. La première étape est la synthèse de l'hydroxyde de zirconium hydraté par co-précipitation d'une solution d'oxonitrate de zirconium $ZrO(NO_3)_2.xH_2O$ (Aldrich, 99%) et d'une solution d'ammoniaque à 28% à pH=8,8. La deuxième étape consiste à stabiliser l'hydroxyde de zirconium hydraté par des espèces siliciques selon la procédure décrite par Nahas et al. (Journal of Catalysis 247 (2007), p51-60). L'hydroxyde de zirconium hydraté est placé dans un ballon en verre contenant une solution ammoniacale dont le pH est ajustée à 11. Le mélange est chauffé à reflux pendant 72h puis filtré et lavé à l'eau permutée. La dernière étape est l'échange entre l'acide tungstique $H_2WO_4$ (Aldrich, 99%) dissous dans du peroxyde d'hydrogène et l'hydroxyde de zirconium. L'acide tungstique est dissous dans une solution de peroxyde d'hydrogène à 35% à 60°C. La concentration de la solution en acide tungstique est de 0,04M. La solution d'acide tungstique est ensuite refroidie à température ambiante, et l'hydroxyde de zirconium dopée à la silice est ajouté lentement. Le solide obtenu est filtré puis calciné sous air à 650°C. Sa surface spécifique est de 40m$^2$/g. Les teneurs en niobium, silicium et zirconium du solide ont été déterminées par ICP-OES. La composition molaire W/Si/Zr de ce catalyseur est 4,7/1,4/93,9.

Exemple 6 : Synthèse du catalyseur H

Le catalyseur H est préparé selon la méthode de synthèse décrite dans l'exemple 1. Le pH de la solution d'acide nitrique est légèrement plus acide (pH<0,1) dans le cas du catalyseur H. Le solide obtenu possède une surface spécifique de 57m$^2$/g et un rapport molaire Zr/Nb de 11,8.

Exemple 7 : préparation et caractérisation du catalyseur B (hors invention)

Le catalyseur ZrTiSiW selon l'invention a été préparé par Rhodia selon la méthode décrite dans le brevet FR2907445A. La surface spécifique de ce catalyseur, déterminée de façon similaire à celle du catalyseur A, est de 105m$^2$/g. La composition en poids d'oxydes de ce catalyseur est 54% de ZrO$_2$, 35% de TiO$_2$, 7,5% de SiO$_2$ et 3,5% de WO$_3$.

Exemple 8 : préparation et caractérisation du catalyseur C (comparatif de l'art antérieur)

Le catalyseur C est une zircone tungstée (89,5% ZrO$_2$-10,5% WO$_3$) synthétisé par Daiichi Kigenso (référence fournisseur : Z-1104). La surface spécifique de ce catalyseur déterminée de façon similaire à celle du catalyseur A est de 77 m$^2$/g.

Exemple 9 : préparation et caractérisation du catalyseur D (comparatif de l'art antérieur)

Le catalyseur D est une zéolithe H-ZSM-5 fournie par Zeochem (ZEOcat PZ-2/5OH). La surface spécifique de ce catalyseur déterminée de façon similaire à celle du catalyseur A est de 406 m$^2$/g.

Exemple 10: Déshydratation catalytique du glycérol en acroléine : évaluation des catalyseurs A, B, C et D

Le tableau 1 donne les performances obtenues avec les catalyseurs A, B, C et D à 6 heures de réaction.

Tableau 1 :

|  | A (invention) | B (hors invention) | C (comparatif) | D (comparatif) |
|---|---|---|---|---|
| Conversion du glycérol | 100 | 100 | 94 | 57 |
| Sélectivité en acroléine | 66 | 69 | 64 | 65 |
| Rendement en acroléine | 66 | 69 | 60 | 37 |
| Rendement en acétaldéhyde | 6,3 | 6,5 | 3,9 | 0,6 |
| Rendement en propionaldéhyde | 3,1 | 5,4 | 2,8 | 1,6 |
| Rendement en acétone | 1,7 | 2,7 | 1,6 | 0,0 |
| Rendement en alcool allylique | 0,1 | 0,5 | 0,5 | 0,2 |
| Rendement en hydroxypropanone | 5,8 | 3,1 | 6,1 | 3,0 |
| Rendement en phénol | 2,6 | 0,8 | 0,3 | - |

Ce tableau montre qu'à volume égal de catalyseur, seul les catalyseurs A et B permettent une conversion totale du glycérol. De plus, les catalyseurs de l'invention permettent d'obtenir une meilleure sélectivité en acroléine, déjà visible à 6 h et qui se confirme à 50 h, avec un rendement en acroléine de 70% pour le catalyseur A et de 80% pour le catalyseur B.
Les catalyseurs A et B sont donc plus actifs et plus sélectifs que les catalyseurs de l'art antérieur.

Exemple 11 : Déshydratation catalytique du glycérol en acroléine : Evolution au cours du temps des performances des catalyseurs A, B, C et D

L'évolution des performances des catalyseurs A, B, C et D au cours du temps, obtenues dans les mêmes conditions que dans l'exemple 4 est présentée sur la figure 1.

Les catalyseurs A et B maintiennent une sélectivité constante en acroléine et une conversion en glycérol élevée sur plusieurs jours contrairement aux catalyseurs C et D de l'art antérieur qui se désactivent fortement en moins de 24h.

Les catalyseurs A et B sont donc plus actifs, plus sélectifs en acroléine mais aussi plus stables dans le temps que les meilleurs catalyseurs revendiqués dans l'art antérieur.

Exemple 12 : Régénération du catalyseur A

Après 143 h sous mélange réactionnel à 300°C, le catalyseur A selon l'invention est régénéré sous flux d'air à 450°C pendant 2h (débit air : 51mL/min). Après régénération, le catalyseur est testé dans les mêmes conditions opératoires qu'avant la régénération.

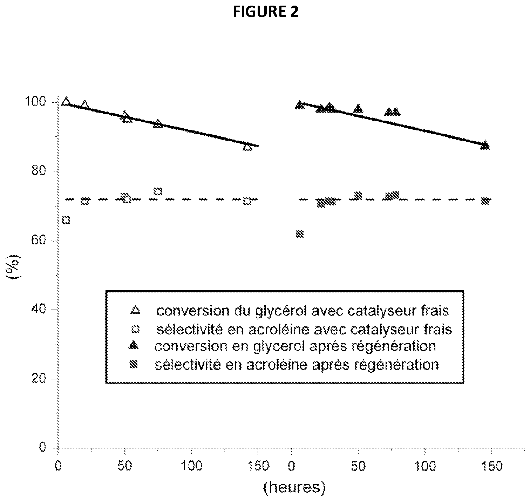

Les résultats obtenus sont présentés sur la figure 2. La régénération sous air à 450°C a permis au catalyseur A de retrouver son activité et son rendement initial. Le catalyseur A selon l'invention est donc régénérable sur un temps court et sans perte d'activité et de sélectivité. Non seulement le catalyseur A est actif et sélectif mais il est également entièrement et facilement régénérable.

Exemple 13 : Déshydratation catalytique du glycérol en acroléine : comparaison des propriétés catalytiques des catalyseurs A', D et C

Le tableau 2 donne les performances obtenues à 300°C avec les catalyseurs A', B et C à 5 heures de réaction.

Tableau 2 :

| | A' (invention) | D (comparatif) | C (comparatif) |
|---|---|---|---|
| Conversion glycérol | 100 | 88 | 99 |
| Rendement en acroléine | 46,8 | 38,8 | 45,6 |
| Sélectivité en acroléine | 47 | 44 | 46 |
| Rendement en acétaldéhyde | 7,9 | 1,3 | 4,6 |
| Rendement en propionaldéhyde | 14,3 | 3,5 | 8,9 |
| Rendement en acétone | 1,4 | 0 | 2,1 |
| Rendement en alcool allylique | 0,9 | 0,5 | 0,5 |
| Rendement en hydroxypropanone | 3,4 | 4,8 | 5,8 |
| Rendement en acide acétique | - | 0,9 | 0,6 |
| Rendement en phénol | 3,4 | 0,2 | 1,3 |

Ce tableau montre qu'à volume égal de catalyseur, seul le catalyseur A' (selon l'invention) permet une conversion totale du glycérol. De plus, le catalyseur A' permet d'obtenir la meilleure sélectivité en acroléine. Le catalyseur A' est donc plus actif et plus sélectif que les catalyseurs de l'art antérieur.

Exemple 14 : Déshydratation catalytique du glycérol en acroléine : Evolution au cours du temps des performances des catalyseurs A', D et C

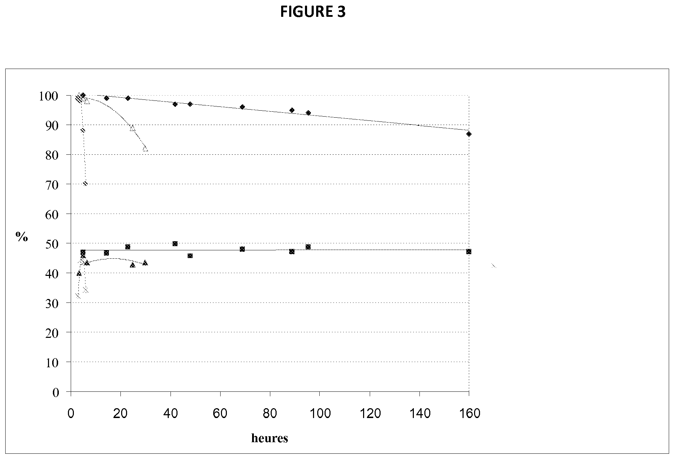

L'évolution des performances des catalyseurs A', D et C au cours du temps est présentée sur la figure 3.

Le catalyseur A' (invention) maintient une sélectivité quasi constante en acroléine et une conversion en glycérol élevée sur une semaine sous flux réactionnel contrairement aux catalyseurs D et C de l'art antérieur qui se désactivent fortement en moins de 24h.

Le catalyseur A' de l'invention est donc plus actif, plus sélectif en acroléine et plus stable dans le temps que les meilleurs catalyseurs revendiqués dans l'art antérieur.

Exemple 15 : Régénération du catalyseur A'

Après 183 h sous mélange réactionnel, le catalyseur A' selon l'invention est régénéré sous flux d'air à 450°C pendant 1h (débit air : 51mL/min). Après régénération, le catalyseur est testé dans les mêmes conditions opératoires qu'avant la régénération.

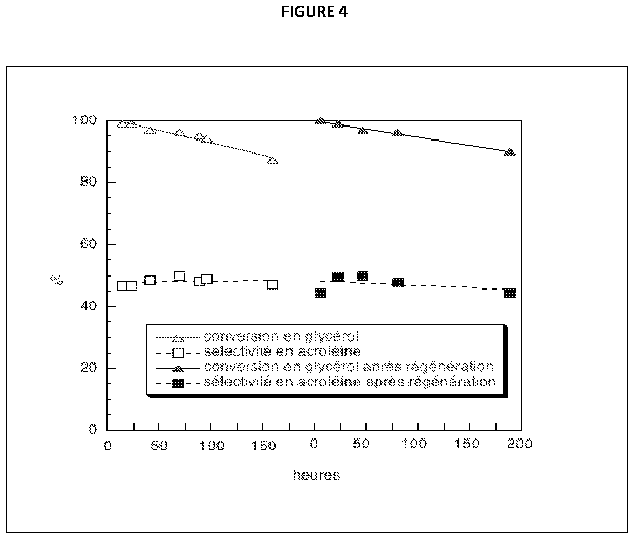

Les résultats obtenus sont présentés sur la figure 4.

La régénération sous air à 450°C a permis au catalyseur A' de retrouver son activité et son rendement initial. Le catalyseur A' selon l'invention est donc régénérable sur un temps court et sans perte d'activité et de sélectivité. Non seulement le catalyseur A' est actif et sélectif mais il est également entièrement et facilement régénérable.

Exemple 16: Déshydratation catalytique du glycérol en acroléine : évaluation des catalyseurs E et F (selon l'invention)

Le tableau 3 donne les performances des catalyseurs E et F obtenues.

Tableau 3 :

|  | E | | | | | F | |
|---|---|---|---|---|---|---|---|
| heure de fin du prélèvement | 5 | 20 | 48 | 72 | 95 | 6 | 24 |
| Conversion du glycérol | 100 | 98 | 97 | 94 | 90 | 100 | 94 |
| Sélectivité en acroléine | 60 | 72 | 73 | 72 | 71 | 50 | 51 |
| Rendement en acroléine | 60 | 71 | 71 | 68 | 63 | 50 | 48 |
| Rendement en acétaldéhyde | 4,9 | 3,1 | 2,6 | 2,6 | 2,5 | 8 | 5,5 |
| Rendement en propionaldéhyde | 6,8 | 4,8 | 3,9 | 3,9 | 3,8 | 5,8 | 4 |
| Rendement en acétone | 1,9 | 1,7 | 1 | 1,1 | 0,9 | 4,1 | 3,2 |
| Rendement en alcool allylique | 0,6 | 0,7 | 0,7 | 0,7 | 0,7 | 3,2 | 4,4 |
| Rendement en hydroxypropanone | 5,1 | 12,2 | 13,5 | 13,1 | 12,4 | 3,1 | 7,7 |
| Rendement en phénol | 1,9 | 0,9 | 0,5 | 0,5 | 0,3 | 1,3 | 0,7 |

Exemple 17: Déshydratation catalytique du glycérol en acroléine : évaluation du catalyseur G

Le tableau 4 donne les performances du catalyseur.

Tableau 4 :

| heures de fin du prélèvement | 4 | 23 | 42 |
|---|---|---|---|
| Conversion du glycérol | 98 | 96 | 87 |
| Sélectivité en acroléine | 68 | 80 | 83 |
| Rendement en acroléine | 67 | 77 | 72 |
| Rendement en acétaldéhyde | 4,2 | 3,5 | 2,4 |
| Rendement en propionaldéhyde | 3,1 | 2,4 | 1,6 |
| Rendement en acétone | 1,2 | 1,3 | 0,9 |
| Rendement en alcool allylique | 0,7 | 0,9 | 0,6 |
| Rendement en hydroxypropanone | 5,2 | 10,9 | 9,7 |
| Rendement en phénol | 0,8 | 0,2 | - |

Exemple 18 : Obtention de l'acroléine à partir de glycérol non pur avec le catalyseur H

Les performances du catalyseur H ont été évaluées avec une solution de glycérine industrielle, brute, titrant 82% en poids. Cette glycérine est caractérisée en ce qu'elle contient plus de 15% en poids de méthanol. Comme dans les exemples précédents, le volume de catalyseur dans le réacteur est de 4,5mL, le débit d'azote est de 74,5mL/min et la température de réaction est de 300°C. Le débit de la solution aqueuse à 20% en poids de glycérine est de 3,77g/h. La proportion relative molaire glycérol/eau/azote est de 1,9/ 46,5/ 51,6. Les résultats obtenus sont donnés dans le tableau 5.

Tableau 5 :

| heure de fin du prélèvement | 8 | 26 | 51 | 76 | 100 | 172 |
|---|---|---|---|---|---|---|
| Conversion du glycérol | 100 | 100 | 100 | 100 | 99 | 90 |
| Sélectivité en acroléine | 56 | 71 | 73 | 73 | 73 | 75 |
| Rendement en acroléine | 56 | 71 | 73 | 73 | 72 | 68 |
| Rendement en acétaldéhyde | 7,4 | 6,2 | 5,4 | 4,6 | 3,9 | 2,8 |
| Rendement en propionaldéhyde | 5,2 | 3,6 | 3,1 | 2,8 | 2,4 | 1,9 |
| Rendement en acétone | 2,0 | 2,0 | 1,4 | 1,2 | 0,9 | 0,6 |
| Rendement en alcool allylique Rendement en | 0,9 | 1,3 | 1,4 | 1,5 | 1,5 | 1,5 |
| hydroxypropanone | 1,9 | 11,1 | 14,5 | 15,6 | 17,9 | 17,5 |
| Rendement en phénol | 5,0 | 1,7 | 0,9 | 0,6 | 0,4 | 0,2 |

La présence d'une quantité importante de méthanol n'abaisse pas les performances du catalyseur de l'invention.

**Revendications**

1. Procédé de préparation d'acroléine à partir de glycérol, ou de glycérine, **caractérisé en ce qu'**on réalise la déshydratation du glycérol ou glycérine en présence d'un catalyseur à base d'oxyde de zirconium et consistant en au moins un oxyde mixte de zirconium et d'au moins un métal M, le dit métal étant le niobium.

2. Procédé selon la revendication 1, **caractérisé en ce que** le catalyseur consiste en au moins un oxyde mixte de zirconium, de niobium et de vanadium.

3. Procédé selon la revendication 1 ou 2, **caractérisé en ce que** l'oxyde dudit catalyseur est supporté.

4. Procédé selon l'une quelconque des revendications 1 à 3, **caractérisé en ce que** le rapport molaire Zr/somme des éléments Si, Ti et M, différents de Zr varie de 0,5 à 200.

5. Procédé selon la revendication 4, **caractérisé en ce que** ledit rapport molaire varie de 1 à 100.

6. Procédé selon l'une quelconque des revendications 1 à 5, **caractérisé en ce que** le glycérol est en solution aqueuse, en une concentration d'au moins 1% en poids.

7. Procédé selon la revendication 6, **caractérisé en ce que** la concentration de la solution aqueuse en glycérol varie de 10 à 50% en poids.

8. Procédé selon l'une quelconque des revendications 1 à 7, **caractérisé en ce que** le catalyseur est régénéré.

9. Procédé de fabrication de l'aldéhyde-3-(méthylthio)propionique MMP, du 2-hydroxy-4-méthylthiobutyronitrile HMTBN, de la méthionine, de l'acide 2-hydroxy-4-méthylthiobutanoïque HMTBA, des esters de ce derniers, ou du 2-oxo-4-méthylthiobutanoïque KMB, à partir d'acroléine, **caractérisé en ce que** l'acroléine est obtenue par un procédé selon l'une quelconque des revendications 1 à 8.

10. Procédé selon l'une des revendications 1 à 9, **caractérisé en ce que** la réaction de déshydratation est réalisée en phase gazeuse.

11. Procédé selon la revendication 10, **caractérisé en ce que** la réaction de déshydratation est réalisée dans un réacteur à lit fixe, à lit fluidisé ou à lit fluidisé circulant.

12. Procédé selon l'une des revendications 1 à 9, **caractérisé en ce que** la réaction de déshydratation est réalisée en phase liquide.

13. Utilisation d'un catalyseur consistant en au moins un catalyseur tel que défini dans l'une quelconque des revendications 1 à 5 et éventuellement 8, pour convertir du glycérol ou de la glycérine en acroléine.

**Patentansprüche**

1. Verfahren zur Herstellung von Acrolein aus Glycerol oder Glycerin, **dadurch gekennzeichnet, dass** das Dehydrieren des Glycerols oder Glycerins in Gegenwart eines Katalysators ausgeführt wird, der auf Basis von Zirkoniumoxid ist und in mindestens einem Mischoxid aus Zirkonium und mindestens einem Metall M besteht, wobei das Metall Niob ist.

2. Verfahren nach Anspruch 1, **dadurch gekennzeichnet, dass** der Katalysator in mindestens einem Mischoxid aus Zirkonium, Niob und Vanadium besteht.

3. Verfahren nach Anspruch 1 oder 2, **dadurch gekennzeichnet, dass** das Oxid des Katalysators getragen wird.

4. Verfahren nach einem der Ansprüche 1 bis 3, **dadurch gekennzeichnet, dass** das Molverhältnis Zr/Summe der Elemente Si, Ti und M, die sich von Zr unterscheiden, von 0,5 bis 200 variiert.

5. Verfahren nach Anspruch 4, **dadurch gekennzeichnet, dass** das Molverhältnis von 1 bis 100 variiert.

6. Verfahren nach einem der Ansprüche 1 bis 5, **dadurch gekennzeichnet, dass** das Glycerol in wässriger Lösung in einer Konzentration von mindestens 1 Gewichts-% vorliegt.

7. Verfahren nach Anspruch 6, **dadurch gekennzeichnet, dass** die Konzentration der wässrigen Lösung an Glycerol von 10 bis 50 Gewichts-% variiert.

8. Verfahren nach einem der Ansprüche 1 bis 7, **dadurch gekennzeichnet, dass** der Katalysator regeneriert wird.

9. Verfahren zur Fabrikation von Aldehyd-3-(methylthio)propionsäure MMP, 2-Hydroxy-4-methylthiobutyronitril HMTBN, Methionin, 2-Hydroxy-4-Methylthiobutansäure HMTBA, Estern dieser letzteren, oder 2-Oxo-4-Methylthiobutansäure KMB, aus Acrolein, **dadurch gekennzeichnet, dass** das Acrolein über ein Verfahren nach einem der Ansprüche 1 bis 8 erhalten wird.

10. Verfahren nach einem der Ansprüche 1 bis 9, **dadurch gekennzeichnet, dass** die Dehydrierungsreaktion in gasförmiger Phase ausgeführt wird.

11. Verfahren nach Anspruch 10, **dadurch gekennzeichnet, dass** die Dehydrierungsreaktion in einem Festbett-, Wirbelbett- oder zirkulierenden Wirbelbettreaktor ausgeführt wird.

12. Verfahren nach einem der Ansprüche 1 bis 9, **dadurch gekennzeichnet, dass** die Dehydrierungsreaktion in flüssiger Phase ausgeführt wird.

13. Verwendung eines Katalysators, der in mindestens einem Katalysator wie in einem der Ansprüche 1 bis 5 und gegebenenfalls 8 definiert, besteht, um Glycerol oder Glycerin in Acrolein umzuwandeln.

**Claims**

1. A method for preparing acrolein from glycerol or glycerin, **characterized in that** the dehydration of glycerol or glycerin is carried out in the presence of a zirconium oxide-based catalyst and consisting of at least one mixed zirconium oxide and at least one metal M, said metal being niobium.

2. The method according to claim 1, **characterized in that** the catalyst consists of at least one mixed zirconium, niobium and vanadium oxide.

3. The method according to claim 1 or 2, **characterized in that** the oxide of said catalyst is supported.

4. The method according to any one of claims 1 to 3, **characterized in that** the molar ratio Zr / sum of elements Si, Ti and M, different from Zr ranges from 0.5 to 200.

5. The method according to claim 4, **characterized in that** said molar ratio ranges from 1 to 100.

6. The method according to any one of claims 1 to 5, **characterized in that** the glycerol is in aqueous solution, in a concentration of at least 1% by weight.

7. The method according to claim 6, **characterized in that** the concentration of the aqueous solution in glycerol ranges from 10 to 50% by weight.

8. The method according to any one of claims 1 to 7, **characterized in that** the catalyst is regenerated.

9. The method for manufacturing 3-(methylthio)propionaldehyde MMP, 2-hydroxy-4-methylthiobutyronitrile HMTBN, methionine, 2-hydroxy-4-methylthiobutanoic acid HMTBA, esters thereof, or 2-oxo-4-methylthiobutanoic acid KMB, from acrolein, **characterized in that** the acrolein is obtained by a method according to any one of claims 1 to 8.

10. The method according to any of claims 1 to 9, **characterized in that** the dehydration reaction is carried out in the gas phase.

11. The method according to claim 10, **characterized in that** the dehydration reaction is carried out in a fixed bed, fluidized bed or circulating fluidized bed reactor.

12. The method according to any of claims 1 to 9, **characterized in that** the dehydration reaction is carried out in the liquid phase.

13. A use of a catalyst consisting of at least one catalyst as defined in any one of claims 1 to 5 and optionally 8, for converting glycerol or glycerin into acrolein.

FIGURE 1

**FIGURE 2**

## FIGURE 3

**FIGURE 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2006087083 A
- WO 2006087084 A
- WO 2007132926 A
- WO 2008066079 A
- US 2008183013 A1
- US 20080214384 A1

- JP 2007268363 A
- JP 2008266165 A
- FR 2907444
- FR 2907445
- FR 2907445 A

### Littérature non-brevet citée dans la description

- **M. PAGLIARO et al.** *Angew. Chem. Int. Ed.,* 2007, vol. 46, 4434-4440
- **M. PAGLIARO ; M. ROSSI.** The Future of Glycerol. *RSC Publishing, Cambridge,* 2008

- **KANTCHEVA et al.** *Catalysis Communications,* 2008, vol. 9 (5), 874-879
- **NAHAS et al.** *Journal of Catalysis,* 2007, vol. 247, 51-60